# EUROPEAN PATENT APPLICATION

(11) **EP 2 253 950 A1**
(43) Date of publication of application: **24.11.2010**
(21) Application number: 10163102.6
(22) Date of filing: 18.05.2010
(51) Int. Cl.: G01N 27/30, G01N 27/403

(54) **Fast response electrochemical organophosphate sensor**

(30) Priority: 19.05.2009 US 468769
(71) Applicant: Honeywell International Inc., Morristown, NJ 07962 (US)
(72) Inventor: Zheng, Zhi, 200030 Shanghai Shanghai (CN); Zhao, Linan, 201204 Pudong New Area Shanghai (CN); Wang, Marilyn, 201203 Pudong New Area Shanghai (CN)
(74) Representative: Buckley, Guy Julian

(57) **Abstract**

The application describes an electrochemical sensor (10) for detecting especially organophosphate (OP) pesticides, eg. parathion. The sensor has a working electrode (12) comprising a three dimensional ordered macroporous (3DOM) TiO₂ layer (14), optionally including electrically conductive material, eg. chitosan. Also described is a method for manufacturing the working electrode.

## Description

### Technical Field

The disclosure relates to electrochemical sensors. More particularly, the disclosure relates to electrochemical sensors for sensing organophosphates.

### Background

A wide variety of electrochemical sensors are known for sensing various chemicals. Of the known electrochemical sensors, each has certain advantages and disadvantages. There is an ongoing need to provide alternative electrochemical sensors as well as alternative methods for manufacturing such electrochemical sensors, including organophosphate sensors.

### Summary

The disclosure relates generally to electrochemical sensors, and more particularly to working electrodes, electrochemical sensors that have a working electrode, and methods for manufacturing the same.

In one illustrative embodiment, an electrochemical sensor may include a porous working electrode. The porous working electrode may, in some cases, be able to detect an organophosphate material having, for example, a nitrobenzene ring. An example organophosphate sensor may include, for example, a porous working electrode that includes TiO₂, sometimes in combination with chitosan to enhance the electrical conductivity of the working electrode. The sensor may also include a counter electrode packaged with the working electrode. A first lead may be attached to the working electrode, and a second lead may be attached to the counter electrode.

In some cases, the working electrode may include a porous Three-Dimensionally Ordered Macro-Porous (3DOM) structure. The Three-Dimensionally Ordered Macro-Porous (3DOM) structure may define pores that are, for example, greater than 100 nanometers but less than 2000 nanometers, but other sizes may be used. In some cases, the pores may have a diameter of about 325 nanometers, but again, this is not required. In some cases, such a Three-Dimensionally Ordered Macro-Porous (3DOM) structure may increase the effective surface area of the working electrode and allow the fluent under test to relatively easily flow between pores of the working electrode, both of which may contribute to increasing the sensitivity and/or reducing response time of the resulting sensor. An example method for manufacturing such working electrode may include providing a substrate, depositing a plurality of nanospheres on the substrate, coating the substrate with a mixture of titanium-containing material and an electrically conductive material, and removing the nanospheres.

The above summary is not intended to describe each disclosed embodiment or every implementation of the present invention. The Figures and Description which follow more particularly exemplify certain illustrative embodiments.

### Brief Description of the Drawings

The disclosure may be more completely understood in consideration of the following description of various illustrative embodiments in connection with the accompanying drawing, in which:
Figure 1 is a schematic side view of an example electrochemical sensor; and
Figure 2 is a schematic side view of a portion of an illustrative working electrode.

While the disclosure is amenable to various modifications and alternative forms, specifics thereof have been shown by way of example in the drawing and will be described in detail. It should be understood, however, that the intention is not to limit the invention to the particular illustrative embodiments described. On the contrary, the intention is to cover all modifications, equivalents, and alternatives falling within the spirit and scope of the invention.

### Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result).

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawing. The drawings, which are not necessarily to scale, depict illustrative embodiments and are not intended to limit the scope of the invention.

Pesticides (e.g., insecticides) are used throughout the world and desirably improve the yield of a variety of crops. Some of the pesticides that are used include organophosphates. Organophosphates, while being effective for controlling insects, may be associated with undesirable health effects for farmers who use the pesticides and for consumers that ingest the pesticides on unwashed or under washed fruits and vegetables. Detection of residual pesticides such as organophosphates on farm produce may be desirable for a number of reasons. For example, the detection of organophosphates may alert sellers and distributors of these products to take appropriate actions in order to remove the chemicals.

Organophosphate detection may also be useful in other industries and situations. For example, organophosphates may find utility in other industries such as in factories, plants, manufacturing facilities, and the like. It may be desirable for these industries to detect organophosphates within the facility as well as in the surrounding areas (e.g., ground water, air, waste water, etc.). Similarly, regulatory agencies may be interested in detecting organophosphates so as to ensure compliance with established rules or guidelines.

Chromatography (e.g., gas chromatography, liquid chromatography, etc.) is a common detection technique that is used to sense the presence of organophosphates. While chromatography is suitable for detection, the process may be time consuming and may involve bulky or cumbersome equipment. Alternative detection systems may include the use of biosensors that utilize enzymes to detect chemicals. These systems, however, may also be time consuming, may only work at certain temperature, and may only be used a single time (i.e. single use).

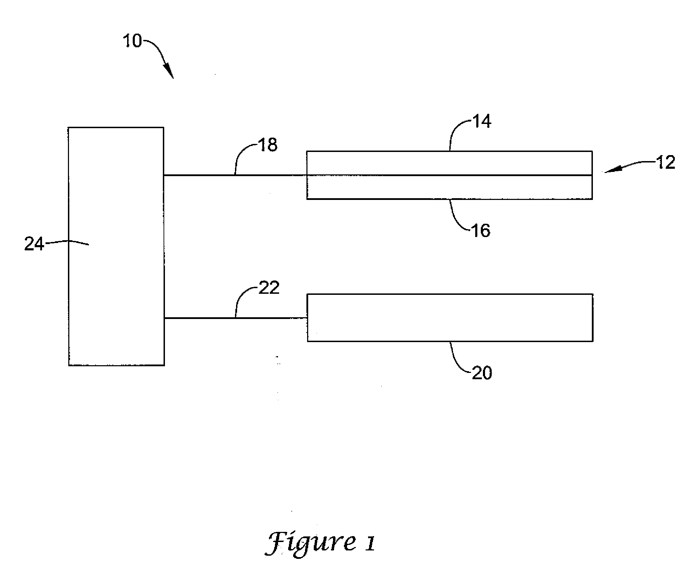

The electrodes and sensors disclosed herein are designed to aid in the detection of chemicals including, for example, organophosphates. An example sensor 10 is schematically depicted in Figure 1. Sensor 10 may be an electrochemical sensor (and/or electrochemical system) that is configured to sense and detect one or more chemicals such as, for example, organophosphates. Sensor 10 may include a working or sensing electrode 12. In some embodiments, working electrode 12 may include a porous layer 14, sometimes disposed on or adjacent to a substrate 16. In some cases, electrode 12 may include a porous layer 14 (i.e., porous layer 14 may form electrode 12), and lack a substrate 16. As shown in the illustrative embodiment of Figure 1, a lead 18 may be coupled to working electrode 12. Sensor 10 may also include an auxiliary or counter electrode 20, with a second lead 22 attached to the counter electrode 20. Leads 18/22 may be coupled to a detector (e.g. meter) 24 for detecting current and/or voltage that is generated between electrodes 12/20 as a result of sensing the presence and/or concentration of a target chemical or class of chemicals in a fluent under test.

Sensor 10 may include other structures and components, if desired. For example, sensor 10 may include a reference electrode (not shown), which may have a lead attached thereto. Alternatively, or in addition, sensor 10 may include an electrolyte (not shown), with one or more of the electrodes in contact with the electrolyte. It is contemplated that electrodes 12/20 (and any additional electrodes and/or electrolyte(s) if present) may be housed in a suitable container or housing. In some cases, the housing may include an opening so that a fluent of interest can enter the sensor 10 and be sensed by electrode 12. It can be appreciated that a wide variety of arrangements are contemplated for sensor 10, which may include a variety of different structures and structural arrangements typical of electrochemical sensors.

In one illustrative embodiment, porous layer 14 may include a material that has a high affinity for organophosphates. In some embodiments, porous layer 14 includes TiO₂. This material may be desirable, for example, because it as able to detect a number of organophosphates. For example, TiO₂ may be able to detect organophosphates including those that have a nitrobenzene structure such as parathion, paraoxon, methyl parathion, fenitrothion, etc. In some cases, porous layer 14 may also include an electrically-conductive material and/or a material that enhances the electrical conductivity of the porous layer 14. For example, porous layer 14 may also include chitosan. In one example, porous layer 14 includes a mixture of TiO₂ and chitosan.

In some cases, porous layer 14 may be structured so as to provide efficient detection of one or more chemicals such as organophosphates. For example, porous layer 14 may be structured as a Three-Dimensionally Ordered Macroporous (3DOM) layer of material that forms all or part of electrode 12. The 3DOM structure may have an inverse opal architecture and may include a TiO₂ backbone. The 3DOM structure may define pores that are, for example, greater than 100 nanometers but less than 2000 nanometers, but other sizes may be used. In some cases, such a 3DOM structure may increase the effective surface area of the working electrode 12 and allow the fluent under test to relatively easily flow between pores of the working electrode, both of which may contribute to increasing the sensitivity and/or response time of the resulting sensor.

The TiO₂ backbone may be or otherwise form an interconnecting network of TiO₂ granules. In some cases, the TiO₂ granules may have a size of about 20-30nm, which may help further increase the surface area of porous layer 14. This architecture may allow electrode 12 to have a relatively large surface or contact area to a fluent of interest. In some cases, the contact area may be 10 to 100 times larger or more relative to a planar surface.

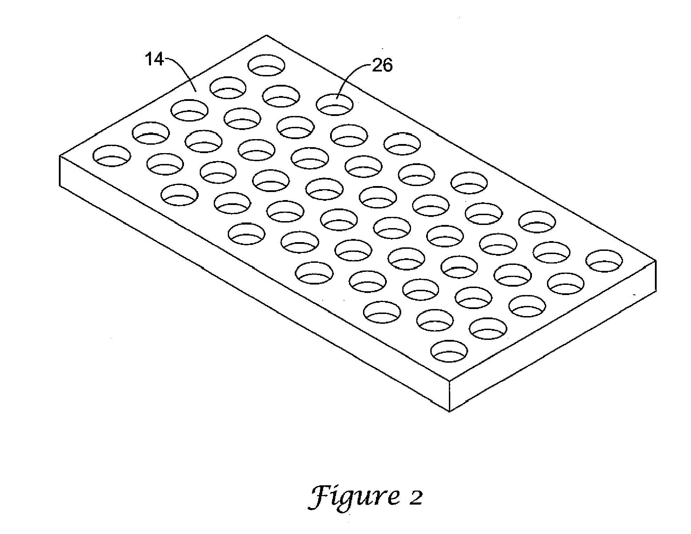

As the name suggests, porous layer 14 may have a plurality of pores 26 formed therein as schematically depicted in Figure 2. Pores 26 may be arranged within layer 14 in any suitable manner. For example, pores 26 may be closely or tightly packed within layer 14, for example in a face-centered cubic structure. Other structures and/or arrangements are contemplated. The pore diameter may be sized to improve sensitivity and/or response time of the sensor 10. For example, for an organophosphates sensor, the diameter of pores 26 may be about 200-400nm, or about 300-350nm, or about 325nm.

Depending on the application, pores 26 of this size, as opposed to nanoporous or mesoporous structures that might have a pore size on the order of about 50nm or less, may allow fluids to more freely flow in and around working electrode 12. Because of this, a sample of a fluent of interest has a greater chance of reaching a greater portion of the surface area of the working electrode 12. Additionally, pores 26 of this size may allow for a sample to more freely and quickly flow around and through electrode. This may not only increase the detection ability of the sensor 10, but may also allow the electrode 12 to be more efficiently and effectively washed so that additionally measurements can be made in a timely manner.

In use, a sample may be provided in contact with working electrode 12. The sample may simply be a fluid that is collected from a location where the presence and/or concentration of an organophosphate is to be monitored and/or detected. Alternatively, the sample may be a wash collected from washing the surface of a particular product (e.g., fresh produce) or a portion of a sample product (e.g., a portion of a piece of fresh produce) that is mixed with an appropriate wash or solution. Regardless of the form of the sample, or how the sample is collected, the user may place the sample in contact with working electrode 12. The sample fluid may be allowed to flow in an around working electrode 12.

If the sample contains the chemical being monitored (e.g., an organophosphate), the material of working electrode 12 may react and create a current and/or voltage, which can be measured by detector 24. Sensor 10 is generally configured and calibrated in a manner such that a current and/or voltage generated at working electrode 12 can be directly correlated to a concentration of the chemical being monitored. Accordingly, and in some cases, sensor 10 can not only senses the presence of a chemical being measured, but also the concentration of the chemical in the sample. Prior or after measuring a sample, working electrode 12 may be washed with a suitable wash solution, as needed. It is contemplated that the sample of the fluent of interest may be a liquid or a gas, depending on the application.

Manufacturing sensor 10 may include any of a number of manufacturing steps. In one example, a number of nanospheres or structures may be applied to or otherwise deposited on a substrate such as a substrate 16. In this example, it is contemplated that substrate 16 may include any material suitable for having the nanospheres deposited thereon. For example, substrate 16 may include a low-iron glass, fluoride- doped tin oxide glass, indium tin oxide glass, any other suitable glass, an electrically conductive material, or any other suitable material as desired. The nanospheres may be made from a variety of different materials. For example, the nanospheres may be polymeric. One suitable polymer that may be used for the nanospheres may be polystyrene. Other materials are also contemplated. The method for depositing the nanospheres on substrate 16 may generally correspond to the material being used for the nanospheres.

In some embodiments, the size (e.g., diameter) of the nanospheres may approximate the desired pore diameter of pores 26. For an organophosphate sensor, the diameter of the nanospheres may be about 200-400nm, or about 300-350nm, about 325nm, or about 300nm. These are only examples, and it is contemplated that any suitable diameter nanospheres may be used. The depositing of the nanospheres on substrate 16 may form a direct opal structure. This structure may vary in thickness, for example, depending on the concentration of the nanosphere material and/or the quantity applied. The direct opal structure may, in essence, act as a mold for forming porous layer 14, as further described below.

A layer of material (e.g., which may ultimately form porous layer 14) may then be applied over the nanospheres and the pores or space formed between the nanospheres. In some cases, the material may include a titanium-containing material. For example, the material may include a titanium precursor such as titanium tetraisopropoxide. Alternatively, the titanium-containing material may include a dispersion of TiO₂ particles. The layer of material may also include an electrically-conductive material or material to enhance the electrical conductivity of the porous layer 14. For example, the layer of material may include chitosan. Thus, in some cases, the layer of material may include a mixture of a titanium-containing material and chitosan, such as a 1:2 mass ratio of the titanium-containing material and the chitosan. Other ratios are also contemplated.

After the layer of material is applied to the nanospheres, the nanospheres may be removed. Removal may include a number of differing processes. For example, removing the nanospheres may include etching away the nanospheres with tetrahydrofuran (i.e., THF etching), benzene, or chloroform. Other processes may also be used, depending on the materials used. Once the nanospheres are removed, the result is the formation of a working electrode 12 including layer 14 having the desired 3DOM structure.

Working electrode 12 may then be packaged in an appropriate manner so as to be configured for use with an end user. For example, electrode 12 may be packaged with a counter electrode 20. Both electrodes 12/20 may have leads 18/22 attached thereto (e.g., via welding).

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A working electrode (12) for an organophosphate sensor (10), the working electrode (12) comprising:
a porous layer (14) including a titanium-containing material, wherein the porous layer (14) has a Three-Dimensionally Ordered Macro-Porous (3DOM) structure, the porous layer (14) suitable for detecting an organophosphate material having a nitrobenzene ring.

2. The working electrode (12) of claim 1, wherein the porous layer (14) includes TiO₂.

3. The working electrode (12) of any one of claims 1-2, wherein the porous layer (14) includes an electrically conductive material.

4. The working electrode (12) of claim 3, wherein the electrically conductive material includes chitosan.

5. The working electrode (12) of any one of claims 1-4, wherein the porous layer (14) includes a plurality of pores (26) having a diameter of about 300nm to 350 nm.

6. An organophosphate sensor (10) comprising:
a porous TiO₂ based working electrode (12) having a Three-Dimensionally Ordered Macro-Porous (3DOM) structure;
a counter electrode (20);
a first lead (18) attached to the working electrode (12); and
a second lead (22) attached to the counter electrode (20).

7. The organophosphate sensor (10) of claim 6, wherein the working electrode (12) includes chitosan.

8. The organophosphate sensor (10) of any one of claims 6-7, wherein the working electrode (12) includes a plurality of pores (26) having a diameter of about 300nm to 350 nm.

9. The organophosphate sensor (10) of any one of claims 6-8, further comprising a reference electrode and a third lead coupled to the reference electrode.

10. A method for manufacturing a working electrode (12) for an organophosphate sensor (10), the method comprising:
providing a substrate (16);
depositing a plurality ofnanospheres on the substrate (16);
coating the substrate (16) with a mixture of titanium-containing material and an electrically conductive material; and
removing the nanospheres.
